# EUROPEAN PATENT APPLICATION

(11) **EP 1 396 275 A1**
(43) Date of publication of application: **10.03.2004**
(21) Application number: 02388061.0
(22) Date of filing: 09.09.2002
(51) Int. Cl.: A61M 5/145

(54) **Visual indicator means for delivery device**

(71) Applicant: NOVO NORDISK A/S, 2880 Bagsvaerd (DK)
(72) Inventor: Pedersen, Per Elgaard, 4690 Haslev (DK)

(57) **Abstract**

The present invention relates to a flow restrictor arrangement suitable for the controlled transfer of fluid from a first compartment to a second compartment. More specifically, the invention relates to indicating means allowing a user to detect an operational state of a delivery device incorporating a flow restrictor. The present invention provides a delivery device comprising a housing, a first cavity containing a drive fluid, a flow restrictor comprising a flow channel, a second cavity in fluid communication with the first cavity through the flow channel, a drug reservoir having in a situation of use an outlet means, where the second cavity and the drug reservoir is arranged such that the volume of the drug reservoir diminishes when the volume of the second cavity increases. The delivery device further comprises drive means for expelling the drive fluid from the first to the second cavity through the flow restrictor, whereby drug is expelled from the drug reservoir through the outlet. In accordance with the invention, the housing comprises a transparent portion allowing the content of the second variable volume cavity or the flow restrictor to be viewed from outside the delivery device, wherein the drive fluid is coloured for easy visual verification of its presence in the second variable volume cavity or the flow restrictor.

## Description

### FIELD OF THE INVENTION

The present invention relates to a flow restrictor arrangement suitable for the controlled transfer of fluid from a first compartment to a second compartment. More specifically, the invention relates to indicating means allowing a user to detect an operational state of a delivery device incorporating a flow restrictor. Such delivery devices are suitable in particular for the in situ administration of a therapeutic drug preparation over a prolonged period of time, however, the delivery devices may also be used in areas such as biochemistry, microbiology and chemical analysis.

### BACKGROUND OF THE INVENTION

In the disclosure of the present invention reference is mostly made to the treatment of diabetes by injection or infusion of insulin, however, this is only a preferred use of the present invention.

Diabetes mellitus is the common name for at least 2 different diseases, one characterised by immune system mediated specific pancreatic beta cell destruction (insulin dependent diabetes mellitus (IDDM) or type 1 diabetes), and another characterised by decreased insulin sensitivity (insulin resistance) and/or a functional defect in beta cell function (non-insulin dependent diabetes mellitus (NIDDM) or type 2 diabetes).

The principal treatment of type 1 diabetes is straight forward substitution of the missing insulin secretion, whereas treatment of type 2 is more complicated. More specifically, in early stages of type 2 diabetes treatment a number of different types of drugs can be used, e.g. drugs which increase insulin sensitivity (ciglitazones), decrease hepatic glucose output (e.g. metformin), or reduce glucose uptake from the gut (alfa glucosidase inhibitors), as well as drugs which stimulate beta cell activity (e.g. sulfonylurea/meglitinides). However, the above-described deterioration is reflected in the fact that beta cell stimulators will eventually fail to stimulate the cell, and the patient has to be treated with insulin, either as mono therapy, or in combination with oral medication in order to improve glucose control.

Currently, there are two principal modes of daily insulin therapy, the first mode including syringes and insulin injection pens. These devices are simple to use and are relatively low in cost, but they require a needle stick at each injection, typically 3-4 times or more per day. The second mode is infusion pump therapy, which entails the purchase of a relatively expensive pump, for which reason the initial cost of the pump is a barrier to this type of therapy. Although more complex than syringes and pens, the pump offer the advantages of continuous infusion of insulin, precision in dosing and optionally programmable delivery profiles and user actuated bolus infusions in connections with meals.

Basically the infusion pump comprises means for allowing the contained insulin to be transferred to the body of the patient. These means may take any desirable form providing the desired function, but presently pump arrangements comprising a conveying arrangement connected to the reservoir (i.e. an outlet to be associated with needle infusion means) and including a pressure or suction generating device for feeding the liquid contained in the reservoir by pressure or suction application from the reservoir to the body are preferred for transferring the insulin contained in the reservoir to the patient. In this respect a number of different principles may be utilized, e.g. osmotic pumps as known from for example US patents 4,340,048 and 4,552,561, piston pumps as known from for example US patent 5,858,001, membrane pumps as known from for example US patent 6,280,148, flow restrictor pumps (also known as bleeding hole pumps) as known from for example US patents 2,605,765 and 5,957,895, and gas generating pumps as known from for example US patent 5,527,288, which all in the last decades have been proposed for use in durable (refillable) and/or disposable (prefilled) drug infusion systems. Often two of the above principles are combined in a single pump, e.g. in US patent 2,605,765 a bleeding hole pump is used to drive a piston pump which may thus be characterized as a "secondary" pump. As some of these principles may not be considered to be pumps in the traditional sense, it may be more appropriate to generally describe these devices as delivery means for fluid, however, in the following description the traditional term pump will be used.

Of the above pump principles, the present invention addresses the bleeding hole pump. Basically, this principle provides a means for establishing a flow of a fluid at a desired rate by applying a force to a liquid to thereby force the liquid through a flow restrictor, the flow rate being determined by the applied force, the flow resistance in the flow restrictor and the viscosity of the fluid. For the purpose of expelling a drug from a reservoir, two variants of this principle have been described.

In a first variant the drug to be infused is contained in a reservoir in fluid communication with an outlet through a flow restrictor. When the drug is pressurized by an actuating (driving) force it is forced through the flow restrictor at a rate determined by the applied force, the flow resistance in the flow restrictor and the viscosity of the drug, see for example US patent 5,957,895 which discloses an infusion device in which the driving force is provided by a drug reservoir formed between two Belleville springs, the flow restrictor being provided by the through-going channel of a capillary tube, or WO 02/15965 disclosing an infusion device in which the flow restrictor is in the form of a tortuous serpentine-formed channel established between two members. In the latter the flow resistance is selectable just as a bolus function is provided. Also US patent 5,993,414 discloses an infusion device utilizing a tortuous path flow restrictor.

In a second variant an infusion device comprises a first cavity containing a drive fluid, a flow restrictor comprising a flow channel, a second cavity in fluid communication with the first cavity through the flow channel, and a drug reservoir containing the drug to be infused, where the second cavity and the drug reservoir is arranged such that the volume of the drug reservoir diminishes when the volume of the second cavity increases. Further, drive means for expelling the drive fluid from the first to the second cavity through the flow restrictor is provided, whereby drug is expelled from the drug reservoir. The force-transmitting interface between the second cavity and the drug reservoir could be described as a secondary pump actuated by the drive means. As appears, the drug flow rate will be determined by the applied force, the flow resistance in the flow restrictor and the viscosity of the drive fluid. Advantages of the second variant are that the (delicate) drug does not have to be forced through the narrow flow restrictor and that a drive fluid having a high viscosity can be used thereby allowing a flow restrictor with a smaller flow resistance to be used which will normally be less expensive to manufacture. Examples of the second variant are disclosed in US patents 2,605,765, 4,561,856, 4,744,786 and 4,437,859 as well as German published patent application 25 52 446.

In the above referred infusion devices using the bleeding hole principle, it has been an object to provide a constant infusion rate which has been achieved using force generating means providing a near-constant force, e.g. different forms of springs. However, it is also possible to use the bleeding hole principle in combination with flow rate controlling means as known from the infusion device described in EP 1 177 802, this infusion device comprising processor controlled valve means which opens and closes the drug flow generated using a bleeding hole pump.

Of the above two variants for a bleeding hole pump, the present invention addresses the second variant.

An advantage of the bleeding hole principle is that it can be implemented in a relatively simple and thus inexpensive way, this lending itself to be utilized in devices in which cost is an important factor. An example of a type of device in which low manufacturing costs are of particular relevance would be a prefilled, disposable infusion device.

Consequently, when it is an object to provide an infusion device which can be manufactured cost-effectively, it would in most cases not be desirable to incorporate expensive components such as electronic control means which in combination with display means could be used to provide the user with information in respect of the infusion process, e.g. display means indicating that infusion is in progress or flow sensors providing information as to the amount of drug infused or left in the reservoir.

In this respect US patent 2,605,765 discloses an infusion device comprising a transparent housing allowing the user to view the actual position of the piston expelling the drug. Although the housing is provided with a graduation it would be difficult for the user to identify that an infusion has just started, i.e. at a time when the piston has hardly moved. Correspondingly, it may be difficult at a quick glance to determine whether the piston is in its rearmost or foremost position and thereby is empty or full. The latter situation may be critical if a used, empty device is applied as thus the user would not receive the intended medication. Especially when used by older or otherwise disabled persons, this risk is considered not merely to be theoretical.

### DISCLOSURE OF THE INVENTION

Having regard to the above-identified problems, it is an object of the present invention to provide a fluid delivery device of the type using a flow restrictor in combination with a drive fluid which provides safe and easy to identify whether infusion has started, thereby providing improved safety, and which can be manufactured in a cost-effective manner.

More specifically, the present invention is based on the realization that the component or structure undergoing the greatest initial transformation or change when pump action is initiated would be the best candidates for detecting this invent.

Thus, in a first aspect the present invention provides a delivery device comprising a housing, a first cavity containing a drive fluid, a flow restrictor comprising a flow channel, a second cavity in fluid communication with the first cavity through the flow channel, a drug reservoir having in a situation of use an outlet means, where the second cavity and the drug reservoir is arranged such that the volume of the drug reservoir diminishes when the volume of the second cavity increases. The delivery device further comprises drive means for expelling the drive fluid from the first to the second cavity through the flow restrictor, whereby drug is expelled from the drug reservoir through the outlet. In accordance with the invention, the housing comprises a transparent portion allowing the content of the second variable volume cavity or the flow restrictor to be viewed from outside the delivery device, wherein the drive fluid is coloured (e.g. using a dye) for easy visual verification of its presence in the second variable volume cavity or the flow restrictor. As most liquid drugs are either transparent or milky (such as crystal-containing insulin) any "strong" colour such as red or blue may be used.

By this arrangement it is possible to identify the initial changes in the flow channel and/or the second cavity. For example, in a preferred embodiment the second cavity is substantially collapsed just as the flow channel preferably is substantially empty, this allowing even very small amounts of coloured drive fluid to be identified visually by the user. In case it is deemed necessary to provide the second cavity with an initial amount of a fluid (e.g. to fill any gaps which may otherwise exist between a flexible drug reservoir surrounded by the second cavity), this initial amount of fluid may advantageously be transparent. This would also apply in case the flow channel was pre-filled with a fluid, however, the volume of the flow channel will for most purposes be neglectable such that an initially air-filled flow channel will be acceptable. Indeed, a given infusion pump may also be designed to primarily indicate to the user that infusion has taken place and that the reservoir is empty.

The bleeding hole arrangement incorporating the indicating principle of the invention may be used in combination with different secondary pump arrangements.

In a preferred embodiment the delivery device comprises a reservoir cavity in which the drug reservoir is contained. The drug reservoir comprises a moveable portion, where the space external to the drug reservoir and between the moveable portion of the drug reservoir and the reservoir cavity defines the second cavity. A flexible membrane member may be arranged within the reservoir cavity thereby dividing the reservoir cavity in the drug reservoir and the second cavity. In a further embodiment the reservoir cavity has a generally cylindrical form with a moveable piston being arranged within the reservoir cavity thereby dividing the reservoir cavity in the drug reservoir and the second cavity.

In a further preferred embodiment the infusion device comprises a drug reservoir defined within a generally flexible enclosure arranged within the reservoir cavity thereby dividing the reservoir cavity in the drug reservoir and the second variable volume cavity substantially surrounding the drug reservoir.

For the above secondary pump arrangements the drug reservoir in an initial state preferably takes up substantially the entire volume of the reservoir cavity, the second variable volume cavity in the initial state being substantially fully collapsed which would allow for easy detection of coloured drive entering the second cavity.

The outlet means may be adapted to be brought in fluid communication with external infusion means (e.g. a catheter tubing or transcutaneous access means such as an infusion needle, a flexible infusion cannula or a plurality of micro-penetrators) or may be supplied with these. In the latter case the fluid communication may be established just prior to use, before or after the drug delivery device has been arranged on the user.

An infusion (or delivery) device of the above type may also be manufactured or offered to the user as a system in which individual components are combined with each other to provide an aggregate device. For example, it may be desirable to offer a system comprising a disposable, pre-filled drug unit, a durable drive-force providing unit and a disposable unit comprising the drive fluid and the flow restrictor. For such a system different flow restrictors providing different infusion rates in combination with a given drive-force could be offered.

Correspondingly, in a second aspect the present invention provides a fluid transmitting device comprising a first variable volume cavity containing a drive fluid, a flow restrictor as discussed and described above, a second variable volume cavity in fluid communication with the first variable volume cavity through the flow channel.

As used herein, the term "drug" or "medicament" is meant to encompass any drug-containing flowable medicament capable of being passed through a delivery means such as a hollow needle in a controlled manner, such as a liquid, solution, gel or fine suspension. There is essentially no limitation on the type of liquid drug which can be used with the invention other than to exclude those liquid drugs which would be inappropriate to deliver to the subject in an auto-mated fashion using the infusion device of the invention. Representative drugs include peptides, proteins, and hormones. In the description of the preferred embodiments reference will be made to the use of insulin. Correspondingly, the term "subcutaneous" infusion is meant to encompass any method of infusion into a subject.

### BRIEF DESCRIPTION OF THE DRAWINGS

In the following the invention will be further described with references to the drawings, wherein
fig. 1 shows a perspective view of a first infusion device in an initial state,
fig. 2 shows a perspective view of the infusion device of fig. 1 in an actuated state,
fig. 3 shows a "horizontal" cross-sectional view of the infusion device of fig. 2,
fig. 4 shows a first "vertical" cross-sectional view of the infusion device of fig. 2,
fig. 5 shows a second "vertical" cross-sectional view of the infusion device of fig. 2,
fig. 6 shows in detail a flow restrictor,
fig. 7 shows in detail an infusion needle,
fig. 8 shows a "horizontal" cross-sectional view of an infusion device in an initial state,
fig. 9 shows the infusion device of fig. 8 in an actuated state,
fig. 10 shows the infusion device of fig. 8 in an empty state,
fig. 11 shows a schematic representation of a second infusion device, and
fig. 12 shows a schematic representation of a further infusion device

### DESCRIPTION OF THE PREFERRED EMBODIMENTS

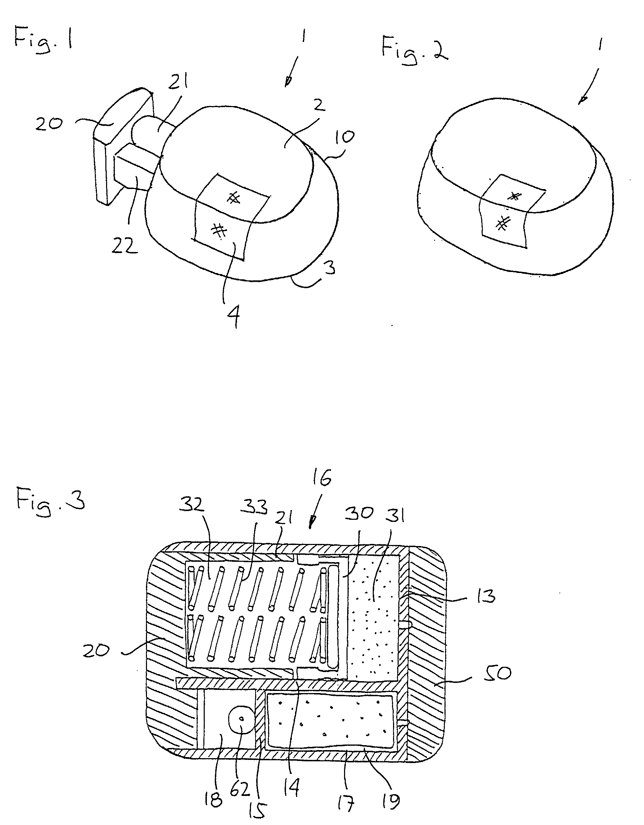

Fig. 1 shows a schematic representation of an embodiment of the invention. Correspondingly, the configuration of the different structures as well as there relative dimensions are intended to serve illustrative purposes only. When in the following terms as "upper", "lower", "right" and "left" or similar relative expressions are used, these only refer to the appended figures and not to an actual situation of use. In the same way the terms "horizontal" and "vertical" refer to planes parallel with respectively perpendicular to a lower surface of the device to be described.

More specifically, fig. 1 shows an infusion device 1 comprising a housing 10 and there from protruding actuation button 20. The housing comprises an upper surface 2 and a lower surface 3 (not to be seen) adapted to be arranged against a skin surface of a user. The upper surface is provided with a transparent window 4 allowing the user to view a drug reservoir arranged within the housing. In fig. 1 the infusion device has been arranged against the skin of a user and the actuation button has pressed into the housing by the user thereby actuating the infusion device as will be explained in detail below.

With reference to figs. 3-5 the general construction of the infusion device will be described. The housing comprises an upper wall 11, a lower planar base plate 12, side wall portions, an end wall 13 with an outer planar surface, and relative to the latter an opposed open end. Internally the housing comprises a first central wall 14 and a second oblique wall 15 in combination defining three compartments, a drive compartment 16, a reservoir compartment 17 and a needle compartment 18. The drive compartment forms a flat cylinder with an open proximal end and a substantially closed distal end. A piston 30 is slidingly arranged in the cylinder dividing the drive compartment in a distal fluid compartment 31 (corresponding to the above-described first cavity) filled with a coloured viscous drive fluid (e.g. silicon oil), and a proximal spring compartment 32. The actuation button 20 comprises a skirt portion 21 slidingly received in the cylinder thereby closing the spring compartment. In the spring compartment are arranged two helical compression springs 33 acting on the piston, however, any compressible material or member providing a spring action or any other means providing or generating a force (e.g. gas generating means or a liquid/gas mixture) acting on the piston may be utilized. The actuation button further comprises a wedge portion 22 to be received in the needle compartment.

As best seen in fig. 5 the reservoir compartment comprises a flexible drug reservoir 40 with an insulin-containing drug formulation. The reservoir is preferably manufactured from a transparent material allowing the user to view and control the drug through the window 4. In the initial state, i.e. before any drug has been expelled from the infusion device, the reservoir has a configuration substantially corresponding to the configuration of the reservoir compartment, thereby forming a neglectable cavity 19 (or dead-space) between the two components. In case an air filled dead space is not acceptable, the space may be filled with a fluid (for illustrative purposes is the gap between the reservoir and the reservoir compartment relatively large). As appears, the dead-space represents the above-described second cavity in a substantially fully collapsed state. Inside the drug reservoir is arranged a U-formed membrane element 41 formed from a self-sealing material and comprising upper and lower membrane portions 42, 43. In the end portion 13 is formed an outlet opening 34 from the fluid compartment and an inlet opening 44 to the reservoir compartment.

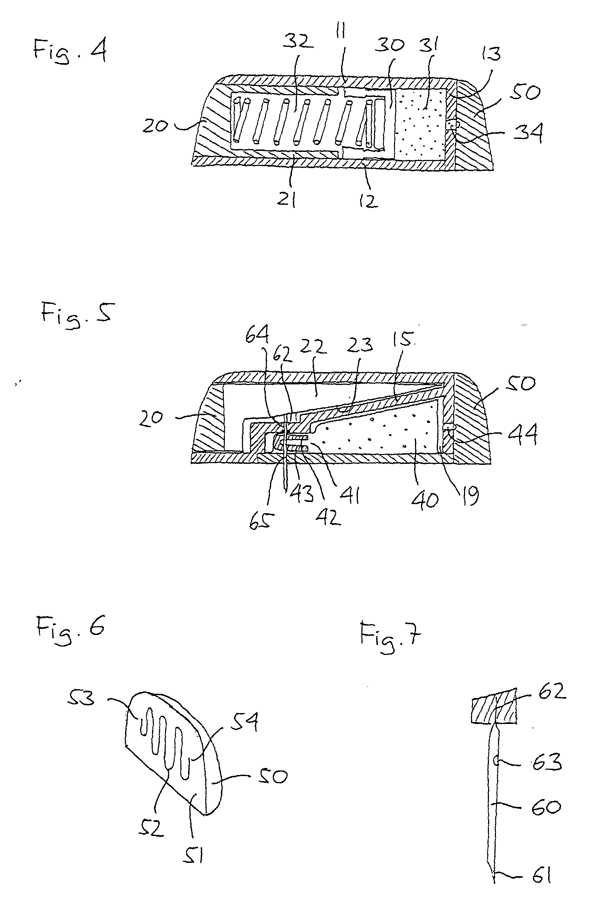

The infusion device further comprises a flow restrictor member 50 (see fig. 6) comprising a planer surface 51 in which a serpentine trace 52 is formed between proximal and distal end portions 53, 54. The flow restrictor member 50 is bonded to the outer planar surface of the housing end portion with the proximal and distal end portions in register with the outlet 34 respectively the inlet openings 44. In this way a flow restrictor channel is formed between the two openings. As appears, the resistance of the flow restrictor, the viscosity of the drive fluid and the force provided by the compressed springs will determine the rate at which the drive fluid will be forced through the flow restrictor to the reservoir compartment. When it is desirable to use the flow restrictor as an indicator means, the flow restrictor member or a portion thereof may be made form a transparent material allowing visual inspection of a coloured drive fluid located in the flow channel.

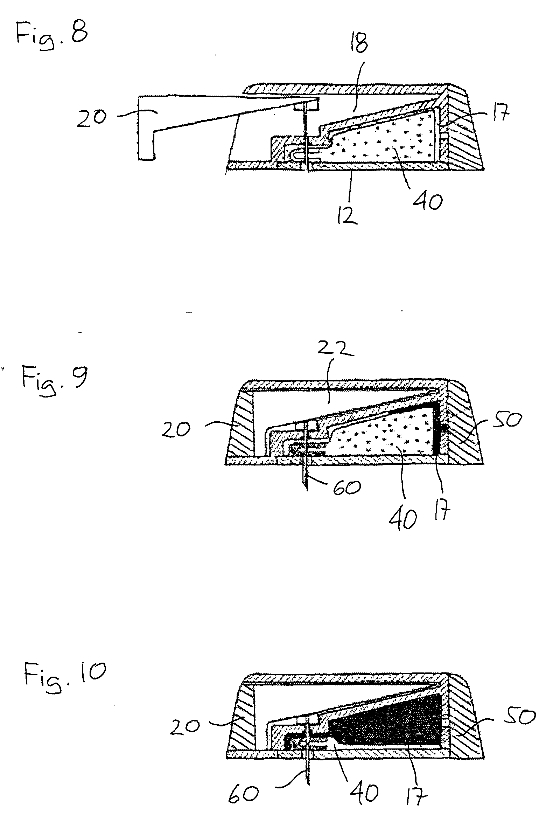

The infusion device further comprises a hollow subcutaneous infusion needle 60 as shown in fig. 7, comprising a distal pointed end 61 adapted to be introduced through a skin surface, a closed proximal end at which a needle wedge 62 is formed. In the body of the needle an opening 63 is formed in flow communication with interior of the needle. The proximal end of the needle is arranged in the needle compartment and with the needle body protruding through an opening 64 formed in the first wall 15 into the reservoir compartment and further into the reservoir. In the initial state (as supplied to the user and not shown in fig. 5) the needle penetrates the upper membrane portion 42 with the distal end 61 arranged between the upper and lower membrane portions 42, 43 inside the reservoir.

Next, with reference to figs. 4 and 5 actuation of the infusion device will be described. When the device has been positioned on a skin surface (preferably the lower surface comprises an adhesive coating) the user actuates the device by fully depressing the actuation button 20 until it locks in place in a recessed position (locking means arranged between the button and the housing is not shown in the figs.) whereby simultaneously the springs 33 are compressed and the wedge portion 22 is moved into the needle compartment. The wedge portion comprises a lower oblique surface 23 in sliding contact with the needle wedge 62 whereby the wedge portion forces the needle downwardly as it is pressed into housing. By this action the pointed distal needle end 61 penetrates the lower membrane portion 43 and is forced out through an opening 65 formed in the base portion. As the infusion device is attached to the skin surface of the user, the infusion needle is hereby introduced through the skin. When the needle is in its fully extended position, the needle opening 63 is positioned between the two membrane portions whereby a fluid communication is established from the drug reservoir to the user. At the same time the drive fluid starts to be expelled from the fluid compartment 31 and through the flow restrictor to the second cavity portion 19 of the reservoir compartment 17 where it gradually will compress the flexible reservoir and thereby force out the therein contained insulin-containing drug through the needle and into the user.

Initially air will be expelled from the needle just as air trapped in the flow restrictor and around the drug reservoir (if any) may result in an initial higher infusion rate, however, these effects will be neglectable.

In the shown embodiment the expelling means in form of springs 33 are "energized" during actuation of the device, however, to reduce the force needed to actuate the button 20 the spring means 33 may be pre-tensioned and the drive fluid 31 correspondingly pre-pressurized, whereby alone puncturing of the reservoir by the needle will actuate the expelling means and thereby start infusion.

Next, with reference to figs. 3 and 8-10 operation of the above-described infusion device will be described, the device comprising a coloured drive fluid which for illustrative purposes is "stronger" coloured than the drive fluid shown in figs. 1-7.

Fig. 8 shows an infusion device in an initial state corresponding to fig. 1, i.e. the actuation button 20 has not yet been pressed into the needle compartment 18 and the reservoir 40 has an initial configuration substantially corresponding to the configuration of the reservoir compartment.

In fig. 9 has the infusion device been attached to a skin surface of a user (not shown) and actuated by depressing the actuation button. As the needle is introduced subcutaneously by means of the wedge portion 22 a fluid communication is established between the user and the drug reservoir. At the same time the springs 33 is compressed whereby the drive fluid starts to be expelled from the fluid compartment 31 and through the flow restrictor to the second cavity portion of the reservoir compartment 17 where it gradually will compress the flexible reservoir and thereby force out the therein contained insulin-containing drug through the needle and into the user. By provision of the window 4 (see fig. 1) the user will be able to detect the initial filling of the space between the reservoir and the walls of the surrounding compartment. In the shown embodiment is the lower surface of the reservoir attached to the base plate 12 which will cause the drive fluid to spread around the upper surface portions of the reservoir corresponding to the window 4. In this way a large area will become coloured early during filling of the second cavity. In addition, the flow restrictor member 50 may be manufactured from a transparent material allowing for even further detection of flow of liquid.

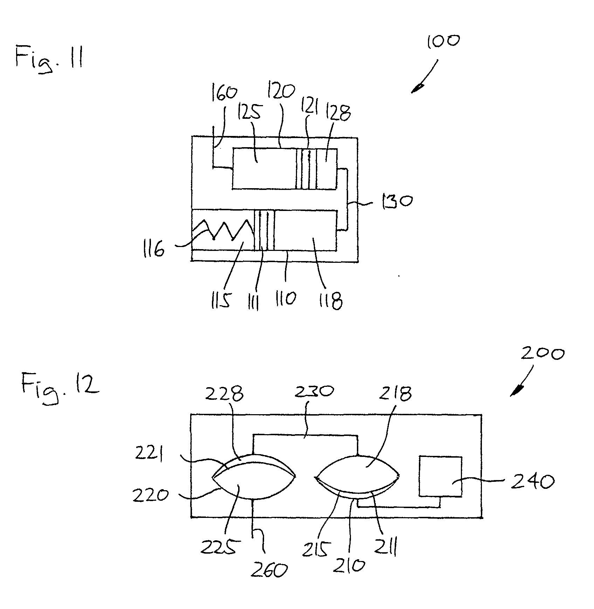

In fig. 10 the reservoir compartment has been filled with drive fluid and the reservoir 40 has correspondingly been emptied, this resulting in an intensely coloured window 4 indicating to the user that the reservoir is empty or substantially empty. When the reservoir as shown is designed to be compressed from above towards the base plate, the "depth" of drive fluid as seen through the window will increase during infusion resulting in a more and more intense colour indicating to the user that infusion is in progress. However, this design does not provide a dosing read-out function and should correspondingly not be used as such.

Depending on the actual design of the infusion device and the arrangement of the flexible drug reservoir, it will be possible to utilize the colour indicating means in different ways. For example, when using a design as described above, the depressed actuation button will clearly indicate that the device has been actuated, however, the colour indicating means will provide the user with additional information as to the state of the infusion device, i.e. that infusion actually has started as indicated by the coloured drive fluid showing up in the flow channel and/or reservoir compartment. In case an actuation means is used which does not allow easy visual confirmation of the state, the colour indicating means may be used to simply indicate that an actual infusion device has been used and should be discarded.

The way the second cavity is filled with drug can be used in different ways depending on the actual design of the reservoir respectively the second cavity.

Fig. 11 shows a schematic representation of a second embodiment of an infusion device comprising first and second cylindrical compartments. The first cylindrical compartment 110 accommodates a first moveable piston 111 dividing the compartment in a drive means cavity 115 and a first cavity 118. The first cavity is filled with a viscous drive fluid and the drive means cavity comprises drive means in the form of a compressed spring 116 exerting a force on the first piston. The second cylindrical compartment 120 forms a reservoir cavity accommodating a second moveable piston 121 dividing the reservoir cavity in a drug reservoir 115 in flow communication with a subcutaneous needle 160 and a second cavity 128 in flow communication with the first cavity through a flow restrictor 130. As the drive fluid is transferred from the first to the second cavity through the flow restrictor, the second piston is forced towards the needle thereby expelling drug in a controlled fashion from the drug reservoir determined by the properties of the force applied on the first piston, the viscosity of the drive fluid and the resistance in the flow restrictor. When a transparent window is provided over the second reservoir cavity the coloured drive fluid makes it easy for the user to identify the position of the second piston and thereby to determine the amount of the drug infused or left.

Fig. 12 shows a schematic representation of a further embodiment of an infusion device comprising first and second compartments. The first compartment 210 accommodates a first flexible membrane 211 dividing the compartment in a drive cavity 215 and a first cavity 218. The drive cavity is in flow communication with drive means 240 comprising a gas/fluid mixture which secures that the drive cavity is supplied with gas at a near-constant pressure thereby exerting a near-constant pressure on the first flexible membrane and thereby the first cavity filled with a viscous drive fluid. The second cylindrical compartment 220 forms a reservoir cavity accommodating a second flexible membrane 221 dividing the reservoir cavity in a drug reservoir 215 in flow communication with a subcutaneous needle 260 and a second cavity 228 in flow communication with the first cavity through a flow restrictor 230. As the drive fluid is transferred from the first to the second cavity through the flow restrictor, the second flexible membrane is forced towards the needle thereby expelling drug from the drug reservoir in a controlled fashion. When a transparent window is provided over the second cavity the coloured drive fluid makes it easy for the user to identify that infusion has started much in the same manner as was the case for the embodiment shown in figs. 8-10.

In the above description of the preferred embodiments, the different structures providing the desired relations between the different components just as the means providing the described functionality for the different components (i.e. force generating means, flow restrictor, flexible reservoir etc.) have been described to a degree to which the concept of the present invention will be apparent to the skilled reader. The detailed construction and specification for the different structures are considered the object of a normal design procedure performed by the skilled person along the lines set out in the present specification.

## Claims

1. A delivery device (1 ) comprising:
- a housing,
- a first variable volume cavity (31 ) containing a drive fluid,
- a flow restrictor (50, 52) comprising a flow channel,
- a second variable volume cavity (19) in fluid communication with the first variable volume cavity through the flow channel,
- a variable volume drug reservoir (40) having in a situation of use an outlet,
- the second variable volume cavity and the variable volume drug reservoir being arranged such that the volume of the drug reservoir diminishes when the volume of the second cavity increases,
- drive means (33) for expelling the drive fluid from the first to the second cavity through the flow restrictor, whereby drug is expelled from the drug reservoir through the outlet,
- the housing comprising a transparent portion allowing the content of the second variable volume cavity or the flow restrictor to be viewed from outside the device,
- wherein the drive fluid is coloured for easy visual verification of its presence in the second variable volume cavity or the flow restrictor.

2. A delivery device as defined in claim 1, comprising a reservoir cavity (17, 120, 220) in which the drug reservoir is contained, the drug reservoir comprising a moveable portion (40, 121, 221 ), the space external to the drug reservoir and between the moveable portion of the drug reservoir and the reservoir cavity defining the second variable volume cavity (19, 128, 228).

3. A delivery device as defined in claim 2, wherein a flexible membrane (221) member is arranged within the reservoir cavity thereby dividing the reservoir cavity in the drug reservoir and the second variable volume cavity.

4. A delivery device as defined in claim 2, wherein the reservoir cavity (120) has a generally cylindrical form, a moveable piston (121) being arranged within the reservoir cavity thereby dividing the reservoir cavity in the drug reservoir (125) and the second variable volume cavity (128).

5. A delivery device as defined in claim 2, wherein the drug reservoir is defined within a generally flexible enclosure (40) arranged within the reservoir cavity (17) thereby dividing the reservoir cavity in the drug reservoir and the second variable volume cavity (19) surrounding at least a portion of the drug reservoir.

6. A delivery device as defined in any of claims 2-5, wherein the drug reservoir in an initial state takes up substantially the entire volume of the reservoir cavity, the second variable volume cavity in the initial state being substantially fully collapsed.

7. A delivery device as defined in any of claims 2-6, wherein the drive fluid comprises a dye.

8. A fluid transmitting device comprising:
- a housing,
- a first variable volume cavity containing a drive fluid,
- a flow restrictor comprising a flow channel,
- a second variable volume cavity in fluid communication with the first variable volume cavity through the flow channel,
- the housing comprising a transparent portion allowing the content of the second variable volume cavity to be viewed from outside the device,
- wherein the drive fluid is coloured for easy visual verification of its presence in the second variable volume cavity.

9. A fluid transmitting device as defined in claim 8 in combination with:
- a variable volume drug reservoir having in a situation of use an outlet,
- the second variable volume cavity and the variable volume drug reservoir being arranged such that the volume of the drug reservoir diminishes when the volume of the second cavity increases, and
- drive means for expelling the drive fluid from the first to the second cavity through the flow restrictor, whereby drug is expelled from the drug reservoir through the outlet.
